# EUROPEAN PATENT SPECIFICATION

(11) **EP 0 687 305 B1**
(45) Date of publication and mention of the grant of the patent: **29.12.1997**
(21) Application number: 94908411.5
(22) Date of filing: 03.03.1994
(51) Int. Cl.: C12P 41/00, C12P 7/52, C12N 1/14, C12N 9/16

(54) **ESTERASE AND ITS USE IN BIOTRANSFORMATION**
ESTERASE UND IHRE VERWENDUNG IN BIOUMWANDLUNG
ESTERASE ET SON UTILISATION EN BIOTRANSFORMATION

(30) Priority: 03.03.1993 GB 9304351
(43) Date of publication of application: 20.12.1995
(73) Proprietor: Chiroscience Limited, Cambridge CB4 4WE (GB)
(72) Inventor: WARNECK, Julie, Belinda, Hazel, Huntingdon PE17 5UU (GB); WISDOM, Richard, Anthony, Cambs. CB4 4NP (GB)
(74) Representative: Perry, Robert Edward
(86) International application number: GB9400408
(87) International publication number: WO9420634

(56) References cited:
- EP-A- 0 195 717
- EP-A- 0 227 078
- EP-A- 0 233 656
- EP-A- 0 407 033
- WO-A-93/04189
- WO-A-93/04190
- WO-A-93/23547

## Description

### Field of the Invention

This invention relates to an esterase and its use in biotransformation.

### Background of the Invention

Many compounds are chiral, and it is often desirable to obtain a substantially pure enantiomer from the racemate that is usually available by conventional chemical synthesis. Such chiral compounds include a number of 2-arylpropionic acids are well known as anti-inflammatory agents. Examples include ibuprofen, naproxen and ketoprofen. It is now well established that their major therapeutic activity resides in the (S)-enantiomer.

Several methods for obtaining the (S)-enantiomer, free of contaminating (R)-enantiomer, are known. These include asymmetric chemical synthesis and chemical resolution such as the stoichiometric crystallisation of diastereomeric salts formed with various chiral amines.

An alternative approach is biocatalytic, using a biocatalyst to selectively hydrolyse an ester of the 2-arylpropionic acid. Providing a biocatalyst with the correct stereo-specificity can be identified, a reaction mixture containing the unreacted ester of one enantiomer plus the acid product of the other enantiomer can be obtained. Separation and recovery of the product are then relatively facile. Unreacted ester can be racemised and reused in a further reaction, thereby ensuring almost complete conversion of the racemic substrate to the required single enantiomer product.

EP-A-0227078 describes the use of several extracellular, commercially-available microbial lipases in such biocatalytic resolutions. In general, however, large amounts of enzyme were required and the reaction took 2-6 days. Such reactions would therefore be expensive to operate. The best enzyme powder identified was that from Candida cylindraceae (also known as Candida rugosa); however, in EP-A-0407033, it was shown that this preparation, as well as having low activity, contained more than one enzyme with esterase activity. Further, in order to obtain ketoprofen with a high ee, it was necessary to purify the preparation.

EP-A-0233656 describes the isolation and cloning of an esterase gene from Bacillus thai. This enzyme is shown to selectively hydrolyse the ethyl and methyl esters of both naproxen and ibuprofen to give the (S)-acid of respective compounds. It is also shown that cloning of the enzyme resulted in a preparation giving a higher ee product, as a result of minimising other enzyme side-activities.

WO-A-9323547 describes a number of strains which also produce an esterase which selectively hydrolyses naproxen esters. The best strain found was Zopfiella latipes, the esterase from which the esterase had been cloned.

WO-A-9304189 describes an organism, Trichosporon sp, that is able to selectively hydrolyse the (S)-enantiomer of ethyl ketoprofen to give an (S)-acid product having an ee of >90%. The enzyme that carries out this biotransformation is intracellular. As stable cell-free preparations have proved difficult to obtain, it is difficult to increase biocatalytic activities by gene cloning or classical mutagenesis.

For economic biotransformation, it is important that cloning techniques should be available, to help reduce enzyme costs. In EP-A-0233656, the relative specific activity of the esterase in the wild Bacillus isolate was very low; thus, even after cloning and hyper-expression of the enzyme, biocatalyst costs are still likely to be significant.

An object behind the present invention has been to obtain a biocatalyst which has good activity against esters, which gives good ee acid product, and which would be suitable for cloning and hyper-expression, e.g. in E. coli, to enable biocatalyst costs to be kept to a minimum.

### Summary of the Invention

Surprisingly, a screen of microorganisms from a range of different sources showed that the ascomycete Ophiostoma novo-ulmi (also known as Ceratocystis ulmi) produced an intracellular hydrolytic enzyme having the desired activity. Further tests demonstrated that, unlike the strain disclosed in WO-A-9304189, stable cell-free activity could be obtained and that therefore it was suitable for cloning and potential use in cell-free biotransformations. Ophiostoma novo-ulmi is a plant pathogen known to be the virulent causative agent of Dutch Elm disease. It is known to be closely related to less virulent strains of Ophiostoma ulmi and also to O. piceae.

Following the primary discovery underlying the present invention, it has been found that stereo-specific esterase activity is present in alternative strains of Ophiostoma novo-ulmi in Ophiostoma ulmi (also sometimes known as Ceratocvstis ulmi) and Ophiostoma piceae (also sometimes known as Ceratocystis piceae) and from isolates collected from various locations in the USA, Europe, the Middle East and Uzbekistan. In addition, alternative strains of Ceratocystis sp. obtained from the IMI, Egham, Surrey, UK have been obtained and screened for activity. It has been discovered that the activity is also present in strains C. coronata (e.g. IMI 176533), C. ips (e.g. IMI 212114), C. tetropii (e.g. IMI 212117), C. cainii (e.g. IMI 176523), C. arborea (e.g. IMI 176529) and C. stenoceras (e.g. IMI 268494). The activity appears therefore to be widespread amongst a range of alternative Ceratocystis strains collected from different locations in the world.

A strain of the original isolate screened, herein described as AJ3, was deposited at the IMI on 15th February 1993, with the accession number IMI 356050, under the terms of the Budapest Treaty. This strain provides a good example of the stereo-specific activity; however, given the widespread nature of the activity in a wide variety of related strains, the scope of the invention is not intended to be limited to this particular organism. The invention also extends to the use of enzymatic activity isolated from such organisms, by any suitable technique. The enzyme per se, or a fusion protein, including the active sequence, may for example be obtained.

### Description of the Invention

In accordance with the present invention, microorganisms of the genus Ophiostoma and their enzymatic activity can be used to hydrolyse the racemic ethyl ester of 2-arylalkanoic acids such as naproxen stereo-selectively, to yield the acid substantially enriched in the (S)-enantiomer, e.g. to 93-96% enantiomeric excess, or more, and leave residual ester enriched with the (R)-enantiomer.

Other substrates include compounds in which the alcohol function is chiral, e.g. esters of 2-methoxycyclohexanol with an alkanoic acid. The novel esterase is thus of wide applicability, although its efficacy on any given substrate can readily be determined by simple experiment. Depending on the nature of the substrate, the (R) or (S)-acid or alcohol may be produced, in admixture with the (S) or (R)-residual ester, respectively.

The reaction can be conducted on any mixture of enantiomers of the ester, although that mixture will usually be the racemate. The reaction is preferably conducted at pH 8-11, more preferably 9.5-10.5, and most preferably about 10. A solvent, e.g. an organic solvent such as cyclohexane, may be employed.

Use of the biocatalyst described is beneficial for the production of chiral alcohols, acids or esters of high enantiomeric purity. Standard chemical procedures may be used to separate out the desired product and, where possible, to racemise for reuse the unwanted enantiomer. In the case where the acid or alcohol formed during the biotransformation has the desired stereochemistry, it is usual to stop the biotransformation at less than 50% conversion to ensure maximal enantiomeric purity of the hydrolytic products. In cases where the residual ester has the required stereochemistry, it is usual to proceed with the biotransformation to greater than 50%. Indeed, in cases where the ester substrate is such that the available biocatalysts have low enantiospecificity, it is generally desirable to select a biocatalyst which preferentially hydrolyses the ester with the incorrect stereochemistry, so leaving the residual ester as the desired product. In this way, it is possible to greatly increase the enantiomeric excess (ee) of the ester product by taking the biotransformation to greater than 50% conversion. Whilst such procedures are known to those skilled in the art, there is clearly some benefit in having access to a biocatalyst with an alternative specificity to those more usually used. As demonstrated in Example 3, the esterase described by the present invention not only has good stereo-specific activity, but also has shown an opposite specificity to a large range of commercially-available enzyme preparations.

The invention has been described in terms of the use of a non-immobilised biocatalyst catalysing hydrolytic reactions. However, in carrying out biotransformations using hydrolytic enzymes, such as the esterase described herein, a number of alternative methodologies are well known to those skilled in the art. Thus, it is now well established that some economic advantage may sometimes be gained by immobilising the enzyme, in any number of ways, thus facilitating enzyme recovery, reuse and, in some instances, stability. Likewise under reaction conditions of lower water activity, it is possible to use such biocatalysts to carry out synthetic, transesterification or interesterification reactions. Such considerations are a matter for conventional process development and optimisation.

The following Examples illustrate the invention.

### Example 1

The following medium was used for the growth of the organism:

| | |
|---|---|
| (NH₄)₂SO₄ (g/l) | 0.5 |
| MgSO₄.7H₂O (g/l) | 0.25 |
| CaCl₂.2H₂O (g/l) | 0.1 |
| KH₂PO₄ (g/l) | 8.0 |
| Yeast Extract (g/l) | 10.0 |
| Glucose (g/l) | 5.0 |
| Trace element solution (µl/l) | 100 |
| pH (with NaOH) | 6.5 |

The trace element solution used was:

| | |
|---|---|
| CaCl₂.2H₂O (g/l) | 3.57 |
| ZnO (g/l) | 2.0 |
| CUCl₂.2H₂O (g/l) | 0.85 |
| Na₂MoO₄.2H₂O (g/l | 4.8 |
| MnCl₂.4H₂O (g/l) | 2.0 |
| FeCl₃.6H₂O (g/l) | 5.4 |
| H₃BO₄ (g/l) | 0.3 |
| CoCl₂.6H₂O (g/l) | 2.4 |
| HCl (ml/l) | 250 |

Cells of AJ3 were inoculated into 100 ml medium in a 1 litre shake flask. This culture was grown with shaking at 30°C for 48 hours. 10 ml was then transferred into 90 ml fresh medium in a 1 litre shake flask and grown for a further 8 hours at 30°C with shaking. This flask was used to inoculate a 2.8 1 laboratory fermenter with 1.5 1 medium plus 5 g/l glucose and 0.5 ml/l of a silicone/PPG-based antifoam (XFO371™, Ivanhoe Chemicals, I11., USA). This fermenter was operated for 48 hours with agitation and aeration, controlled to maintain a DOT >50% air saturation and temperature and pH at 30°C and 6.0 respectively.

At the completion of the fermentation the cells were harvested by centrifugation and the pellet resuspended at 10% w/v (based on wet cell weight) in lysis buffer, i.e. 0.1M sodium carbonate in 5% Triton™ X-100 aqueous solution. Lysis was carried out overnight at 8°C with shaking after which the enzyme activity-containing lysate was separated from the cell debris by centrifugation. The lysate was then diluted 50% in lysis buffer, prior to its being used to carry out resolution.

Racemic ethyl naproxen (ethyl 6-methoxy-α-methyl-2-naphthaleneacetate) was dissolved in cyclohexane to a concentration of 19 mg/ml. 2 ml of this solution was then mixed with 5 ml of prepared crude enzyme solution in a glass vial and reacted with shaking for 72 hours at 23°C.

Analysis of the aqueous phase of the reaction mixture after 72 hours showed accumulation of naproxen to 2.76 mg/ml, equivalent to about 40% conversion. HPLC analysis of the enantiomeric purity of this naproxen showed it to have an ee (enantiomeric excess) of 93% in favour of the (S) enantiomer (equivalent to 96.5% pure).

### Example 2

This Example illustrates the cloning of the stereo-specific esterase from the isolate AJ3, and the use of the product.

### Isolation of poly(A)+ m-RNA

10 g of filtered AJ3 mycelium was ground under liquid nitrogen into a fine powder. The total genomic RNA was then isolated from the ground mycelium using the acid guanidinium thiocyanate-phenol-chloroformextractionmethod as detailed by Chomczynski & Sacchi, Anal. Biochem. 162:156-159 (1987). This method yielded 8.76 mg/ml of total genomic RNA with a 260:280 nm ratio of 1.704.

The poly(A)⁺ m-RNA was isolated from the total genomic RNA by affinity chromatography on oligo (dT)-cellulose as detailed by Sambrook et al in Molecular Cloning, A Laboratory Manual, 2nd Ed., Cold Spring Harbor Laboratory Press (1989). Specifically, 0.5 g of oligo (dT)-cellulose was resuspended in 10 ml of 0.1 M NaOH, and 1 ml of this slurry was poured into a 2.5 ml sterile disposable syringe barrel. A packed bed volume of 0.8 ml was achieved. 10.5 mg of total genomic RNA was allowed to bind to the equilibrated oligo (dT)-cellulose. The genomic RNA was eluted from the column using excess volumes of column loading buffer. Progress was monitored spectrophotometrically, the eluate being analysed at 260 nm. When there was no appreciable absorbance at 260 nm, the poly(A)⁺ m-RNA was eluted using a suitable elution buffer. Progress was monitored spectrophotometrically. The samples were pooled and then reapplied to the oligo (dT)-cellulose, and the elution effected again. The final samples yielded 85 µg/ml of high purity poly(A)⁺ m-RNA.

### Preparation of a c-DNA library

The c-DNA library was constructed using the ZAP-cDNA™ synthesis kit (Stratagene Ltd., La Jolla, CA, USA). 8.5 µg of the poly(A)⁺ m-RNA were used at the onset of the protocol. The efficacies of the first and second syntheses were analysed by autoradiography. Following spin column gel filtration chromatography through Sephacryl™ S-400, the size-fractionated c-DNA samples were quantified using ethidium bromide-impregnated agarose; values in excess of 10 ng/µl were measured. High molecular weight c-DNA samples were ligated into the uni-ZAP XR vector, and then packaged into E. coli XL1-Blue MRF' using the Gigapack™ II packaging extracts (Stratagene Ltd.). The titre of the resultant c-DNA library was 1.63 x 10¹⁰ plaque-forming units (pfu)/ml.

### Screening of the c-DNA library for esterase activity

Sufficient recombinant phage were used, to yield 50,000 pfu against E. coli XL1-Blue MRF' when cultivated on 150 mm NZY agar plates. 1.2 x 10⁶ recombinant phage were allowed to develop under inducing conditions (i.e. in the presence of 2.5 mM IPTG). The phage were screened for esterase activity using a 0.1% w/v S(+) ketoprofen ethyl ester agarose overlay. Esterase activity was indicated by conversion of the poorly water soluble ethyl ester to soluble ketoprofen acid. After an overnight incubation at 24°C, 8 discrete clearance zones were noted. The plaques associated with the clearance zones were cored and the phage recovered. The esterase-positive phage were reamplified in E. coli XL1-Blue MRF', retitred and screened for esterase activity, using both the substrate overlay method and by HPLC.

### In vivo excision of the recombinant p-Bluescript from Uni-ZAP-XR

The recombinant p-Bluescript phagemid located within the Uni-ZAP XR vector was excised from the vector using the ExAssist/SOLR™ system (Stratagene Ltd.). 62 recombinant phagemids were rescued and inherited stably in E. coli SOLR. All of these recombinant E. coli were screened for esterase activity using the substrate overlay method and proved to be positive. A comparison of colony diameter versus substrate clearance zone diameter indicated that strain numbers 47 and 62 were worthy of further study, and were designated CS47 and CS62, respectively.

### Restriction digest map of recombinant plasmids

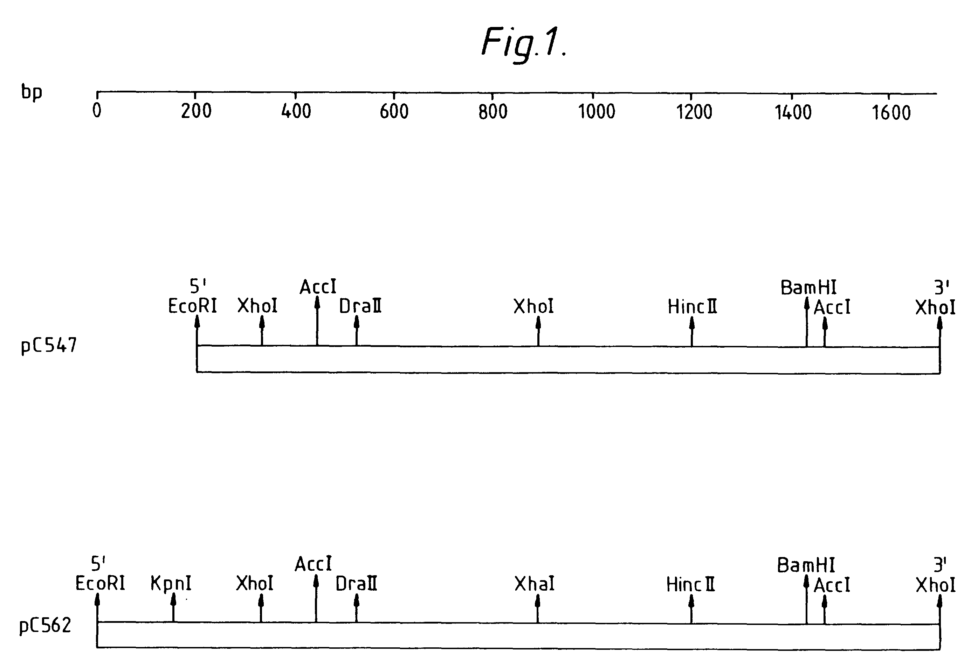

400 ml LB plus ampicillin (50 µg/ml) cultures of recombinant E. coli XL1-Blue MRF' strains CS47 and CS62 were grown. Plasmids (designated pCS47 and pCS62, respectively) were prepared from them by the method of Holmes & Quigley (Anal. Biochem. 114:193 (1981)), and purified by ultracentrifugation in CsCl-ethidium bromide gradients. These were then subjected to a number of restriction endonuclease digestions, using commercially-available enzymes and under the suppliers conditions. Fragment sizes were assessed by agarose gel electrophoresis, different percentages of agarose being used to confirm fragment sizes. Results of the restriction digest are shown in Figure 1. Notably, pCS47 was approximately 200 base pairs shorter than pCS62 at the 5' end.

### Growth of recombinant strain

Recombinant strain CS62 was inoculated into 100 ml LB medium plus 50 µg/ml ampicillin in a 1 litre shake flask. This was incubated for 15 hours at 37°C with shaking (orbital shaker, 25 mm throw) at 300 rpm. This was then inoculated at 1% into 1.5 litre medium in a 2.8 litre laboratory fermenter. The following medium was used:

| | |
|---|---|
| Tryptone (Unipath Ltd., Hants., UK) | 12.0 g/l |
| Yeast extract (Unipath Ltd.) | 24.0 g/l |
| Glycerol | 4.0 ml/l |
| KH₂PO₄ | 2.3 g/l |
| K₂HPO₄.3H₂O | 16.4 g/l |

The temperature was maintained at 25°C, the pH at 7.0 by automatic addition of 10% phosphoric acid as required, and the dissolved oxygen tension >50% of air saturated by agitation control. An air flow rate of 0.75 l/min was used. Growth was monitored by measurement of sample absorbances at 520 nm against a medium blank after suitable dilutions. At an absorbance of 0.9, IPTG (isopropyl-β-D-thiogalactoside) was added to a final concentration of 240 µg/ml.

After 24 hours growth in the fermenter, the cells were harvested by centrifugation and stored at -20°C until required.

### Use of recombinant strain

Cells of CS62 grown and stored as described above were used to provide enzyme for use in the hydrolysis of ethyl naproxen. Cells were resuspended at 10% w/v in 0.1 M sodium carbonate, pH 10, and lysed by sonication for 10 minutes (10 seconds on, 10 seconds off cycles) at 4°C. The enzyme lysate was then centrifuged to obtain a cell-free supernatant solution.

Racemic ethyl naproxen was dissolved in cyclohexane to a concentration of 100 g/l. 200 µl of this solution was added to 1 ml enzyme solution and shaken for 1 hour at 25°C. The phases were then separated and the aqueous phase analysed (by reverse phase HPLC) for naproxen accumulation. It was found that naproxen had accumulated to 5.3 g/l (about 26% conversion). Analysis showed an ee of >95% in favour of the S-enantiomer (the (R)-enantiomer was so low that it could not reliably be measured).

Analysis of a biotransformation using an enzyme extract from E. coli SOLR (the host non-recombinant strain) showed no activity against the ester.

### Example 3

Cells (grown as per Example 2) were resuspended at 5% w/v in 100 mM TRIS, pH 8, and lysed by sonication for 15 minutes (10 seconds on, 10 seconds off cycle) at an amplitude of 20 µm in a Soniprep™ 150. The lysate was centrifuged to remove cell debris and the supernatant used in biotransformation.

10 g of the racemic butyrate ester of 2-methoxycyclohexanol was mixed with 100 ml of 100 mm TRIS, pH 8, by magnetic stirring. 20 ml of the above lysate of CS62 was added. The temperature of the biotransformation was controlled at 25°C and the pH at 8.0 by the addition of 1 M NaOH. Samples were removed and monitored for 2-methoxycyclohexanol formation. NaCl was added to saturation to 1 ml sample which was then extracted into 1 x 5 volumes of toluene. The toluene layer was removed and dried by the addition of MgSO₄. The sample was then analysed by GC. A 15 m DB-5 column with 0.25 µm film was used with helium as the carrier, at 55.2 kPa, the oven temperature was 60°C for 1 minute, raised at 10°C/min to 150°C, and this temperature was then held for a further minute.

The ee of the 2-methoxycyclohexanol formed was determined following derivatisation with trifluoroacetic anhydride (TFAA). 20 µl of TFAA was added to 1 ml of the toluene-extracted sample which was then allowed to stand at ambient temperature for 30 minutes. The diastereomers were resolved by GC using a 30 m GTA column (Chiraldex™) at 96 kPa, helium as carrier gas and with the oven at 110°C.

After 41 hours reaction, there was 44% conversion of the substrate, with an ee of the 2-methoxycyclohexanol of 96% in favour of the (S)-enantiomer.

The stereo-specific activity of this biocatalyst is considered unusual. A screen of 12 commercially-available lipase/esterase preparations on the above substrate showed none which had this specificity, for the (S)-enantiomer. Thus, commercially-available lipase/esterase preparations from porcine pancreatic lipase, Rhizopus arrhizius, Rhizopus delemar, Mucor miehei, Aspergillus niger, Penicillium cyclopium, Pseudomonas fluorescens all showed specificity for the (R)-enantiomer, whereas preparations from Candida cylindracae, Mucor javanicus and cholesterol esterase showed no specificity. Other preparations from Penicillin roquefortii and Candida lipolytica showed minimal if any activity against the racemic butyrate ester of 2-methoxycyclohexanol. Therefore, none of the above shows high specificity for hydrolysis of the (S)-enantiomer. This is potentially advantageous in that it enables access to the (S)-enantiomer as the alcohol without having to exceed 50% conversion.

## Claims

1. Use of an organism of the genus Ophiostoma or Ceratocystis, or a material having enzymatic activity derived therefrom, as a stereo-specific esterase in the preparation of an enantiomer of a chiral acid, alcohol or ester, other than (S)-ketoprofen, from a mixture of enantiomers of an ester derived from the acid and alcohol.

2. Use according to claim 1, wherein the organism or the said material is characterised by the ability to react stereo-selectively with racemic ethyl naproxen and give (S)-naproxen in at least 93% enantiomeric excess, at 15-25% conversion.

3. Use according to claim 1 or claim 2, wherein the organism has the characteristics of that available from Ophiostoma novo-ulmi, IMI 356050.

4. Use according to any preceding claim, wherein the ester is of a 2-arylalkanoic acid other than ketoprofen.

5. Use according to claim 4, for the production of (S)-naproxen from the racemic ethyl ester thereof.

6. Use according to any of claims 1 to 3, wherein the alcohol is chiral.

7. Use according to any preceding claim, wherein the reaction is conducted at pH 8 to 11.

8. Use according to any preceding claim, wherein the reaction is conducted at pH 9.5 to 10.5.

## Patentansprüche

1. Verwendung eines Organismus der Gattung Ophiostoma oder Ceratocystis oder eines Materials mit davon abgeleiteter enzymatischer Aktivität als stereospezifische Esterase zur Herstellung eines Enantiomers einer(s) chiralen Säure, Alkohols oder Esters, außer (S)-Ketoprofen, aus einer Mischung von Enantiomeren eines Esters, der sich von der Säure und dem Alkohol ableitet.

2. Verwendung nach Anspruch 1, worin der Organismus oder das Material gekennzeichnet ist durch das Vermögen, stereoselektiv mit racemischem Ethylnaproxen zu reagieren und (S)-Naproxen in mindestens 93% enantiomerem Überschuß bei 15-25% Umsatz zu ergeben.

3. Verwendung nach Anspruch 1 oder Anspruch 2, worin der Organismus die Eigenschaften desjenigen aufweist, der von Ophiostoma novo-ulmi, IMI 356050 erhältlich ist.

4. Verwendung nach irgendeinem der vorhergehenden Ansprüche, worin der Ester von einer anderen 2-Arylalkansäure als Ketoprofen stammt.

5. Verwendung nach Anspruch 4 zur Herstellung von (S)-Naproxen aus dessen racemischem Ethylester.

6. Verwendung nach irgendeinem der Ansprüche 1 bis 3, worin der Alkohol chiral ist.

7. Verwendung nach irgendeinem der vorhergehenden Ansprüche, worin die Umsetzung bei pH 8 bis 11 durchgeführt wird.

8. Verwendung nach irgendeinem der vorhergehenden Ansprüche, worin die Umsetzung bei pH 9,5 bis 10,5 durchgeführt wird.

## Revendications

1. Utilisation d'un micro-organisme du genre Ophiostoma ou Ceratocystis ou d'un matériel ayant une activité enzymatique qui en est dérivée, comme estérase stéréospécifique dans la préparation d'un énantiomère d'un acide, alcool ou ester chiral autre que le (S)-kétoprofène, à partir d'un mélange d'énantiomères d'un ester dérivé de l'acide et l'alcool.

2. Utilisation selon la revendication 1, où le micro-organisme ou ledit matériel est caractérisé par sa capacité à réagir de façon stéréosélective avec le naproxène d'éthyle racémique et à donner du (S)-naproxène en un excès d'énantiomère d'au moins 93 %, pour une conversion de 15 à 25 %.

3. Utilisation selon la revendication 1 ou la revendication 2, où le micro-organisme a des caractéristiques qui sont celles du Ophiostoma novo-ulmi disponible, IMI 356050.

4. Utilisation selon l'une quelconque des revendications précédentes, où l'ester est un ester d'acide 2-arylalcanoïque autre que le kétoprofène.

5. Utilisation selon la revendication 4 pour la production de (S)-naproxène à partir de son ester d'éthyle racémique.

6. Utilisation selon l'une quelconque des revendications 1 à 3, où l'alcool est chiral.

7. Utilisation selon l'une quelconque des revendications précédentes, où la réaction est mise en oeuvre à un pH de 8 à 11.

8. Utilisation selon l'une quelconque des revendications précédentes, où la réaction est mise en oeuvre à un pH de 9,5 à 10,5.
